# EUROPEAN PATENT SPECIFICATION

(11) **EP 2 056 832 B1**
(45) Date of publication and mention of the grant of the patent: **22.03.2017**
(21) Application number: 07794121.9
(22) Date of filing: 20.08.2007
(51) Int. Cl.: A61K 31/519, A61K 9/20, A61K 9/48

(54) **COMPOSITIONS, SUITABLE FOR ORAL ADMINISTRATION, COMPRISING A TRIAZOLO [4, 5-D]PYRIMIDIN DERIVATE**
ZUSAMMENSETZUNGEN ZUR ORALEN VERABREICHUNG MIT EINEM TRIAZOLO [4, 5-D]PYRIMIDIN-DERIVAT
COMPOSITIONS POUR ADMINISTRATION ORALE COMPRENANT UN DÉRIVÉ DE TRIAZOLO [4, 5]PYRIMIDINE

(30) Priority: 21.08.2006 US 823083 P
(43) Date of publication of application: 13.05.2009
(73) Proprietor: AstraZeneca AB, 151 85 Södertälje (SE)
(72) Inventor: BANKS, Simon, Loughborough Leicestershire LE11 5RH (GB)
(74) Representative: Berry, Ian Gordon
(86) International application number: PCT/SE2007/000736
(87) International publication number: WO 2008/024045

(56) References cited:
- WO-A1-00/34283
- WO-A1-01/36421
- WO-A1-01/92262
- WO-A1-2004/037263
- WO-A1-2004/052342
- WO-A2-2004/024127
- WO-A2-2005/113006
- US-A1- 2004 001 885

## Description

The present invention relates to pharmaceutical compositions and more particularly to a pharmaceutical composition containing the compound of formula (**I**)**:**

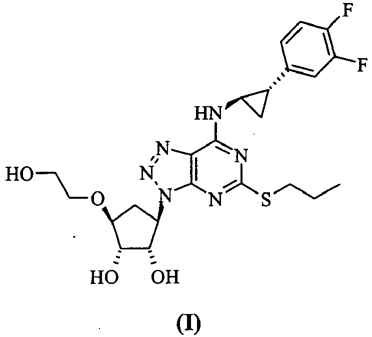

The compound of formula **(I)** is conventionally named {1*S*-[1α,2α,3β(1*S**,2*R**),5β]}-3-(7-{[2-(3,4-difluorophenyl)cyclopropyl]amino}-5-(propylthio)-3*H-*1,2,3-triazolo[4,5-*d*]pyrimidin-3-yl)-5-(2-hydroxyethoxy)cyclopentane-1,2-diol and hereinafter will be referred to as the 'Agent'.

The Agent is disclosed as an ADP-receptor antagonist in International Patent Application number PCT/SE99/02256 (publication number WO00/34283) and International Patent Application number PCT/SE01/01239 (publication number WO01/92262). It has been found that adenosine 5'-diphosphate (ADP) acts as a key mediator of thrombosis. ADP-induced platelet aggregation is mediated by the P*_{2T}* receptor subtype located on the platelet membrane. The P*_{2T}* receptor (also known as P2Y_{ADP} or P2T_{AC}) is primarily involved in mediating platelet aggregation/activation and is a G-protein coupled receptor which is as yet uncloned. The pharmacological characteristics of this receptor have been described, for example, in the references by Humphries et al., Br. J. Pharmacology (1994), 113, 1057-1063, and Fagura et al., Br. J. Pharmacology (1998) 124, 157-164. It has been shown that antagonists at this receptor offer significant improvements over other anti-thrombotic agents (see J. Med. Chem. (1999) 42, 213).

The pharmaceutical compositions of the present invention are suitable for oral administration. One of the qualities that is desirable in a pharmaceutical composition suitable for oral administration is bioavailability. The bioavailability of a drug is the relative amount of an administered dose that reaches the systemic circulation in an unchanged form. Therefore bioavailability is important in determining the therapeutically active concentration at the site of action. Both drug release from the formulation and the stability of the formulation will affect its bioavailability. It is therefore important that the drug formulation should release substantially all of the drug. (see Aulton ME, Pharmaceutics - The Science of Dosage Form Design, 2nd Edition, 2002, Churchill Livingstone.). Bioavailability can be measured using tests know in the art, for example using a standard United States Pharmacopoiea (USP) dissolution apparatus and a standard 'bio-relevant' dissolution medium, for example FaSSIF (Pharm. Res.,17:439-444, 2000).

There are pharmaceutical compositions containing the Agent that retain some of the Agent and hence reduce its bioavailability.

We have now discovered a novel pharmaceutical composition of the Agent that has advantageous properties and which solves one or more of the problems associated with formulation of the Agent. In a first aspect we have discovered a pharmaceutical composition that is suitable for oral administration and that releases substantially all of the Agent. In one aspect the pharmaceutical composition releases at least 90% of the Agent. In another aspect the pharmaceutical composition releases at least 95% of the Agent. In yet another aspect the pharmaceutical composition releases at least 97% of the Agent.

Accordingly, the invention is a pharmaceutical composition comprising:
the Agent;
a filler which is a mixture of mannitol and dibasic calcium phosphate dihydrate,
a binder which is hydroxypropyl cellulose,
a disintegrant which is sodium starch glycollate,
and one or more lubricants.

A 'soluble' filler is a filler that is substantially soluble in water at ambient temperature. An 'insoluble' filler is a filler that has low or slow solubility in water at ambient temperature.

The pharmaceutical composition contains at least one 'soluble' filler which is mannitol.

The pharmaceutical composition contains one or more 'insoluble' filler which is dibasic calcium phosphate dihydrate.

In one aspect the pharmaceutical composition contains one or more 'soluble' fillers. In another aspect, the pharmaceutical composition contains one 'soluble' filler.

In one aspect the pharmaceutical composition contains one or more 'insoluble' fillers. In another aspect, the pharmaceutical composition contains one 'insoluble' filler.

In one aspect the pharmaceutical composition contains one or more binders. In another aspect, the pharmaceutical composition contains one binder.

In one aspect the pharmaceutical composition contains one or more disintegrants. In another aspect, the pharmaceutical composition contains one disintegrant.

In one aspect the pharmaceutical composition contains one or more lubricants. In another aspect, the pharmaceutical composition contains one lubricant.

In another aspect, the filler is a mixture of mannitol and dibasic calcium phosphate dihydrate.

The 'soluble' filler is mannitol.

The 'insoluble' filler is dibasic calcium phosphate dihydrate.

The binder is hydroxypropyl cellulose.

The disintegrant is sodium starch glycollate.

Additional conventional excipients, which may be added, include preservatives, stabilisers, anti-oxidants, silica flow conditioners, antiadherents or glidants.

Other suitable lubricants and additional excipients which may be used are described in Handbook of Pharmaceutical Excipients, 2nd Edition, American Pharmaceutical Association; The Theory and Practice of Industrial Pharmacy, 2nd Edition, Lachman, Leon, 1976; Pharmaceutical Dosage Forms: Tablets Volume 1, 2nd Edition, Lieberman, Hebert A., et al, 1989; Modem Pharmaceutics, Banker, Gilbert and Rhodes, Christopher T, 1979; and Remington's Pharmaceutical Sciences, 15th Edition, 1975.

Suitable lubricants include, for example, magnesium stearate, stearic acid, palmitic acid, calcium stearate, carnauba wax, hydrogenated vegetable oils, mineral oil, polyethylene glycols and sodium stearyl fumarate.

In one aspect, the lubricant is selected from magnesium stearate and sodium stearyl fumarate. In another aspect, the lubricant is magnesium stearate.

In one aspect, the pharmaceutical composition contains from 1 to 50% by weight of the Agent. In particular it contains 20 to 45% by weight of the Agent.

In another aspect, the pharmaceutical composition contains from 1 to 90% by weight of filler. In particular, it contains 20 to 70% by weight of filler.

In another aspect, the pharmaceutical composition contains from 1-70% by weight of 'soluble' filler. In particular, it contains 20 to 45% by weight of 'soluble' filler.

In another aspect, the pharmaceutical composition contains from 1 to 30% by weight of 'insoluble' filler. In particular it contains 10 to 30% by weight of 'insoluble' filler.

In another aspect, the pharmaceutical composition contains from 2 to 8% by weight of binder. In particular, it contains 3 to 6% by weight of binder.

In another aspect, the pharmaceutical composition contains from 2 to 6% by weight of disintegrant.

It will be appreciated that a particular excipient may act as both a binder and a filler, or as a binder, a filler and a disintegrant. Typically the combined amount of filler, binder and disintegrant comprises, for example, 50 to 90% by weight of the composition.

Typically one or more lubricants will be present in an amount 0.5 to 3%, and especially 0.5 to 1% by weight.

The invention relates to a pharmaceutical composition comprising the Agent, mannitol, dibasic calcium phosphate dihydrate, hydroxypropylcellulose, sodium starch glycollate and one or more lubricants.

In another aspect the invention relates to a pharmaceutical composition comprising: the
Agent in an amount of 20 to 45% by weight;
mannitol in an amount of 20 to 45% by weight;
dibasic calcium phosphate dihydrate in an amount of 10 to 30% by weight; hydroxypropylcellulose in an amount of 3 to 6% by weight;
sodium starch glycolate in an amount of 2 to 6% by weight; and
one or more lubricants in an amount of 0.5 to 3% by weight.

It is desirable that the physical properties of these compositions are stable on storage, as changes in for instance, disintegration times, dissolution rates or tablet hardness among others can affect product performance. It is possible that decreases in dissolution rate on storage under International Council for Harmonisation of Technical Requirements for Registration of Pharmaceuticals for Human Use (ICH) stability testing conditions, used to assign product shelf life, can reduce the bioavailability of the Agent. Physical property stability can be measured by USP methodologies for disintegration times and dissolution testing.

It is desirable that the compositions are chemically stable as degradation by oxidation, hydrolysis, isomerisation, photolysis, polymerisation or any other method of degradation, either as a result of mixing with excipients or by any other method, could lead to a reduction in bioavailability. Chemical stability can be measured by a suitable, stability indicating chromatographic method for determining degradation products (see Aulton ME, Pharmaceutics - The Science of Dosage Form Design, 2nd Edition, 2002, Churchill Livingstone.).

In another aspect we have discovered a pharmaceutical composition that is suitable for oral administration that releases substantially all of the Agent and has a desirable stability profile.

In one aspect the invention relates to a pharmaceutical composition prepared by wet granulation.

Granulation is a process by which primary particles (powders) are made to adhere to form larger, multiparticulate entities called granules. Granulation normally commences after initial dry mixing of the powdered ingredients so that a fairly uniform distribution of ingredients through the mix is achieved. Granulation methods can be divided into two types, wet granulation methods that utilize a liquid to form the granules and dry methods that do not.

In dry granulation methods, primary powder particles are aggregated under pressure (or compaction). There are two main processes: a large tablet (also known as a slug) is produced with a heavy duty tablet press or the powder particles are compressed between two rollers to produce a sheet or 'ribbon' of material (process known as roller compaction). In both cases, the compacted material is milled using a suitable milling technique to produce granular material. The granules can then be compressed in a standard tablet press to produce tablets.

Wet granulation involves massing the primary powder particles using a granulating fluid. The fluid contains a solvent, which can be removed by drying, and is non-toxic. The granulating fluid can be used alone or more typically with a binding agent (binder) to ensure article adhesion in the dry state. Binding agents can be added to the system as a binder solution (as part of the granulating fluid) or as dry material mixed with the primary powder particles. There are three main types of wet granulator, shear granulators (such as planetary mixers), high shear mixer granulators (such as Fielder or Diosna) and Fluid Bed Granualtors (such as Aeromatic or Glatt).

In another aspect we have discovered a pharmaceutical composition prepared by a wet granulation process that is suitable for oral administration that releases substantially all of the Agent and a desirable stability profile.

In another aspect the invention relates to a pharmaceutical composition prepared by a wet granulation process comprising the Agent, mannitol, dibasic calcium phosphate dihydrate, hydroxypropylcellulose, sodium starch glycollate and one or more lubricants.

In another aspect the invention relates to a pharmaceutical composition prepared by high shear wet granulation.

High shear wet granulation is a process that involves intensive dry mixing of primary powders and subsequent addition of granulating fluid, which results in the formation of granules. The granulating fluid contains a volatile solvent (usually water) and may also include a binder; ensuring particle adhesion (binders may also be added dry as powders to the bulk of the formulation to be granulated). Granules possess major advantages compared to powders, which they are composed of, in terms of improved flow properties, reduced risk of segregation, increased homogeneity. (Information taken from Aulton ME, Pharmaceutics - The Science of Dosage Form Design, 2nd Edition, 2002, Churchill Livingstone.)

In one aspect the pharmaceutical composition is in a solid dosage form, such as a tablet or capsule. In another aspect the pharmaceutical composition is in the form of a tablet.

In another aspect the invention relates to a pharmaceutical composition prepared by a high shear wet granulation process comprising the Agent, mannitol, dibasic calcium phosphate dihydrate, hydroxypropylcellulose, sodium starch glycollate and one or more lubricants.

The Agent exists in an amorphous form and in four different substantially crystalline forms (see International Patent Application number PCT/SE01/01239 (publication number WO01/92262)). In another aspect the invention relates to a pharmaceutical composition as hereinabove define in which the Agent is in a crystalline form.

In yet another aspect the invention relates to a pharmaceutical composition comprising the Agent substantially as Polymorph II.

In yet another aspect the invention relates to a pharmaceutical composition comprising the Agent substantially as Polymorph III.

Compositions of the invention, which are of particular interest include, for example, the specific embodiments set out hereinafter in the accompanying Example.

It will be appreciated that modifications of the wet granulation techniques, including the order of addition of the components and their screening and blending prior to compression into tablets, may be carried out according to principles well known in the art.

A further aspect of the present invention comprises a method of preparing a pharmaceutical composition, which comprises admixing the Agent with:
a filler which is a mixture of mannitol and dibasic calcium phosphate dihydrate;
a binder which is hydroxyl propyl cellulose,
a disintegrant which is sodium starch glycollate,
and one or more lubricants.

### Example 1

| Ingredient | Quantity per unit dose Unit dose (mg) | Quantity (%w/w or w/v) |
|---|---|---|
| The Agent | 90.00 | 30.00 |
| Mannitol | 126.00 | 42.00 |
| Dibasic calcium phosphate dihydrate | 63.00 | 21.00 |
| Hydroxypropyl cellulose | 9.00 | 3.00 |
| Sodium starch glycollate | 9.00 | 3.00 |
| Magnesium stearate | 3.00 | 1.00 |
| Core tablet weight | 300.000 | 100.00 |

A high shear wet granulator (Fielder GP1 with 10L bowl) was used to dry mix the Agent, mannitol, dibasic calcium phosphate dihydrate, hydroxypropyl cellulose and sodium starch glycolate in amounts to give 2.5 kg of total formulation, for 4 minutes. Water was added via a pressure pot at approximately 50g/min to approx. 25% w/w. The total mixing time was approximately 10 minutes.

The fluid bed was dried using a Glatt GPCG1 at 60°C to a product temperature of 42°C. The resulting granule was milled by Quadro Comil 197, the milled granules were blended with magnesium stearate and tablets were compressed from the blend.

## Claims

1. A pharmaceutical composition comprising:
{11S-[1α,2α,3β(1S*,2R*),5β]}-3-(7-{[2-(3,4-difluorophenyl)cyclopropyl]-amino}-5-(propylthio)-3H-1,2,3-triazolo[4,5-d]pyrimidin-3-yl)-5-(2-hydroxyethoxy)-cyclopentane-1,2-diol;
a filler which is a mixture of mannitol and dibasic calcium phosphate dihydrate;
a binder which is hydroxypropyl cellulose;
a disintegrant which is sodium starch glycollate; and
one or more lubricants.

2. A pharmaceutical composition as defined in claim 1 wherein the lubricant is selected from magnesium stearate and sodium stearyl fumarate.

3. A pharmaceutical composition as defined in claim 1 or 2 wherein {1S-[1α, 2α,3β(1S*,2R*),5β]}-3-(7-{[2-(3,4-difluorophenyl)cyclopropyl]-amino}-5-(propylthio)-3H-1,2,3-triazolo[4,5-d]pyrimidin-3-yl)-5-(2-hydroxyethoxy)-cyclopentane-1,2-diol is present in an amount of 20 to 45% by weight.

4. A pharmaceutical composition as defined in any one of claims 1 to 3 wherein the filler is present in an amount of 20 to 70% by weight.

5. A pharmaceutical composition as defined in any one of claims 1 to 4 wherein the binder is present in an amount of 3 to 6% by weight.

6. A pharmaceutical composition as defined in any one of claims 1 to 5 wherein the disintegrant is present in an amount of 2 to 6% by weight.

7. A pharmaceutical composition as defined in any one of claims 1 to 6 wherein the lubricant is present in an amount of 0.5 to 1 % by weight.

8. A pharmaceutical composition as defined in claim 1 comprising:
{1S-[1α,2α,3β(1S*,2R*),5β]}-3-(7-{[2-(3,4-difluorophenyl)cyclopropyl]amino}-5-(propylthio)-3H-1,2,3-triazolo[4,5-d]pyrimidin-3-yl)-5-(2-hydroxyethoxy)-cyclopentane-1,2-diol in an amount of 20 to 45% by weight;
mannitol in an amount of 20 to 45% by weight;
dibasic calcium phosphate dihydrate in an amount of 10 to 30% by weight;
hydroxypropylcellulose in an amount of 3 to 6% by weight;
sodium starch glycolate in an amount of 2 to 6% by weight; and
one or more lubricants in an amount of 0.5 to 3% by weight.

9. A pharmaceutical composition as defined in claim 1 or 2 wherein the lubricant is magnesium stearate.

## Patentansprüche

1. Pharmazeutische Zusammensetzung, umfassend:
{1S-[1α,2α,3β(1S*,2R*),5β]}-3-(7-{[2-(3,4-Difluorphenyl)cyclopropyl]amino}-5-(propylthio)-3H-1,2,3-triazolo[4,5-d]pyrimidin-3-yl)-5-(2-hydroxyethoxy)cyclopentan-1,2-diol,
einen Füllstoff, bei dem es sich um eine Mischung aus Mannitol und Calciumhydrogenphosphat-dihydrat handelt,
ein Bindemittel, bei dem es sich um Hydroxypropylcellulose handelt,
ein Sprengmittel, bei dem es sich um Natriumstärkeglykolat handelt, und
ein oder mehrere Schmiermittel.

2. Pharmazeutische Zusammensetzung nach Anspruch 1, wobei das Schmiermittel aus Magnesiumstearat und Natriumstearylfumarat ausgewählt ist.

3. Pharmazeutische Zusammensetzung nach Anspruch 1 oder 2, wobei {1S-[1α,2α,3β(1S*,2R*),5β]}-3-(7-{[2-(3,4-Difluorphenyl)cyclopropyl]amino}-5-(propylthio)-3H-1,2,3-triazolo[4,5-d]pyrimidin-3-yl)-5-(2-hydroxyethoxy)cyclopentan-1,2-diol in einer Menge von 20 bis 45 Gew.-% vorliegt.

4. Pharmazeutische Zusammensetzung nach einem der Ansprüche 1 bis 3, wobei der Füllstoff in einer Menge von 20 bis 70 Gew.-% vorliegt.

5. Pharmazeutische Zusammensetzung nach einem der Ansprüche 1 bis 4, wobei das Bindemittel in einer Menge von 3 bis 6 Gew.-% vorliegt.

6. Pharmazeutische Zusammensetzung nach einem der Ansprüche 1 bis 5, wobei das Sprengmittel in einer Menge von 2 bis 6 Gew.-% vorliegt.

7. Pharmazeutische Zusammensetzung nach einem der Ansprüche 1 bis 6, wobei das Schmiermittel in einer Menge von 0,5 bis 1 Gew.-% vorliegt.

8. Pharmazeutische Zusammensetzung nach Anspruch 1, umfassend:
{1S-[1α,2α,3β(1S*,2R*),5β]}-3-(7-{[2-(3,4-Difluor-phenyl)cyclopropyl]amino}-5-(propylthio)-3H-1,2,3-triazolo[4,5-d]pyrimidin-3-yl)-5-(2-hydroxy-ethoxy)cyclopentan-1,2-diol in einer Menge von 20 bis 45 Gew.-%,
Mannitol in einer Menge von 20 bis 45 Gew.-%,
Calciumhydrogenphosphat-dihydrat in einer Menge von 10 bis 30 Gew.-%,
Hydroxypropylcellulose in einer Menge von 3 bis 6 Gew.-%,
Natriumstärkeglykolat in einer Menge von 2 bis 6 Gew.-% und
ein oder mehrere Schmiermittel in einer Menge von 0,5 bis 3 Gew.-%.

9. Pharmazeutische Zusammensetzung nach Anspruch 1 oder 2, wobei es sich bei dem Schmiermittel um Magnesiumstearat handelt.

## Revendications

1. Composition pharmaceutique comprenant :
le {1S-[1α,2α,3β(1S*,2R*),5β]}-3-(7-{[2-(3,4-difluorophényl)cyclopropyl]-amino}-5-(propylthio)-3H-1,2,3-triazolo[4,5-d]pyrimidin-3-yl)-5-(2-hydroxyéthoxy)-cyclopentane-1,2-diol ;
une charge qui est un mélange de mannitol et de phosphate de calcium dibasique dihydraté ;
un liant qui est l'hydroxypropylcellulose ;
un délitant qui est le glycolate d'amidon sodique ; et
un ou plusieurs lubrifiants.

2. Composition pharmaceutique telle que définie dans la revendication 1 dans laquelle le lubrifiant est choisi parmi le stéarate de magnésium et le stéaryl-fumarate de sodium.

3. Composition pharmaceutique telle que définie dans la revendication 1 ou 2 dans laquelle le {1S-[1α,2α,3β(1S*,2R*),5β]}-3-(7-{[2-(3,4-difluorophényl)cyclopropyl]-amino}-5-(propylthio)-3H-1,2,3-triazolo[4,5-d]pyrimidin-3-yl)-5-(2-hydroxyéthoxy)-cyclopentane-1,2-diol est présent en une quantité de 20 à 45 % en poids.

4. Composition pharmaceutique telle que définie dans l'une quelconque des revendications 1 à 3 dans laquelle la charge est présente en une quantité de 20 à 70 % en poids.

5. Composition pharmaceutique telle que définie dans l'une quelconque des revendications 1 à 4 dans laquelle le liant est présent en une quantité de 3 à 6 % en poids.

6. Composition pharmaceutique telle que définie dans l'une quelconque des revendications 1 à 5 dans laquelle le délitant est présent en une quantité de 2 à 6 % en poids.

7. Composition pharmaceutique telle que définie dans l'une quelconque des revendications 1 à 6 dans laquelle le lubrifiant est présent en une quantité de 0,5 à 1 % en poids.

8. Composition pharmaceutique telle que définie dans la revendication 1 comprenant :
du {1S-[1α,2α,3β(1S*,2R*),5β]}-3-(7-{[2-(3,4-difluorophényl)cyclopropyl]amino}-5-(propylthio)-3H-1,2,3-triazolo[4,5-d]pyrimidin-3-yl)-5-(2-hydroxyéthoxy)- cyclopentane-1,2-diol en une quantité de 20 à 45 % en poids ;
du mannitol en une quantité de 20 à 45 % en poids ;
du phosphate de calcium dibasique dihydraté en une quantité de 10 à 30 % en poids ;
de l'hydroxypropylcellulose en une quantité de 3 à 6 % en poids ;
du glycolate d'amidon sodique en une quantité de 2 à 6 % en poids ; et
un ou plusieurs lubrifiants en une quantité de 0,5 à 3 % en poids.

9. Composition pharmaceutique telle que définie dans la revendication 1 ou 2 dans laquelle le lubrifiant est le stéarate de magnésium.
